# EUROPÄISCHE PATENTANMELDUNG

(11) **EP 2 959 931 A1**
(43) Veröffentlichungstag der Anmeldung: **30.12.2015**
(21) Anmeldenummer: 14173786.6
(22) Anmeldetag: 24.06.2014
(51) Int. Cl.: A61M 5/30

(54) **Nadellose Injektionsvorrichtung**

(71) Anmelder: LTS LOHMANN Therapie-Systeme AG, 56626 Andernach (DE)
(72) Erfinder: LELL, Peter, 85368 Moosburg (DE); ANAMUR, Cihad, 81375 München (DE); FELLNER, Christian, 85304 Illmünster (DE)
(74) Vertreter: Simandi, Claus

(57) **Zusammenfassung**

Die Erfindung betrifft eine nadellose Injektionsvorrichtung bzw. eine Vorrichtung zur nadellosen Injektion einer Substanz in ein Gewebe oder Körper, mit welcher vorzugsweise ein Stoff, insbesondere Wirkstoff mittels hohen Drucks injiziert werden kann. Eine erfindungsgemässe Vorrichtung umfasst eine Kammer (11), die ein pyrotechnisches Material enthält, wobei die Kammer an einer Austrittsöffnung eine fixierte Doppelmembran (13, 15) aufweist, wobei die hautseitige Membran (15) der Doppelmembran mit einer Substanzauftragung (25) versehen ist. Eine erfolgreiche Injektion besteht darin, dass genügend Substanz durch die Gewebe-, oder Körperbarriere, insbesondere solche wie die Haut, in den Körper eingebracht wird.

## Beschreibung

Die Erfindung betrifft eine nadellose Injektionsvorrichtung bzw. eine Vorrichtung zur nadellosen Injektion einer Substanz in ein Gewebe oder Körper, mit welcher vorzugsweise ein Stoff, insbesondere Wirkstoff mittels hohen Drucks injiziert werden kann. Eine erfolgreiche Injektion besteht darin, dass genügend Substanz durch die Gewebe-, oder Körperbarriere, insbesondere solche wie die Haut, in den Körper eingebracht wird. Das Grundprinzip, einen Stoff nadellos mittels hohen Drucks zu injizieren, ist seit geraumer Zeit bekannt (siehe z. B. US 3,308,818).

Es sind verschiedene Typen von nadellosen Injektionsvorrichtungen im Stand der Technik beschrieben, wie z. B. nicht abschließend eine Zylinder-Kolben-Einheit umfassend ein vorgespanntes Federelement (DE 10 2007 004 211 A1) oder mittels sogenannter Injektionspatronen (WO 98/31409) als auch mittels einer Gaspatrone zur Beaufschlagung eines Kolbens (US 5,399,163).

Weiterhin können nadellose Injektionsvorrichtungen aus einem pyrotechnischen Antrieb bestehen, der beispielsweise zur Betätigung eines Kolbens genutzt wird und auf diese Weise das Auspressen eines Wirkstoffes aus einer Kanüle erzeugt (EP 1 557 190 A1).

Eine weitere Ausführungsform einer nadellosen Injektionsvorrichtung besteht in einem pyrotechnischen Ansatz, wobei in einer Kammer (auch Brennkammer genannt) eine Explosion erzeugt wird, wobei die freigesetzte Energie zur Impulsübertragung auf eine Membran genutzt wird, wodurch eine membranständige adsorbierte Substanz unter Ablösung von der Membran in Richtung des Gewebes hinreichend beschleunigt wird.

Eine solche gattungsmäßige Vorrichtung ist in WO 2004/071558 A1 offenbart. Es gilt jedoch eine solche gattungsmäßige Ausführungsform zu verbessern. Insbesondere ist gemäß der Lehre von WO 2004/071558 nachteilig, dass die vorgesehene Membran platzen kann und folglich nicht ausgeschlossen ist, dass Teile der Membran als auch die Abgase aus der Brennkammer das Gewebe unter Schädigung des Gewebes bzw. Körpers erreichen.

Von daher nimmt die vorliegende Erfindung Bezug auf eine gattungsmäßige Ausführungsform gemäß WO 2004/071558, jedoch mit der objektiven Aufgabenstellung, das Gewebe oder einen Körper von Membranteilen und Abgasen freizuhalten.

Daher betrifft die vorliegende Erfindung eine nadellose Injektionsvorrichtung umfassend eine (Brenn-)Kammer (11) enthaltend ein pyrotechnisches Material, wobei die Kammer (11) an einer Austrittsöffnung eine fixierte Doppelmembran (13, 15) aufweist, wobei die hautseitige Membran (15) der Doppelmembran mit einer Substanzauftragung (25) versehen ist.

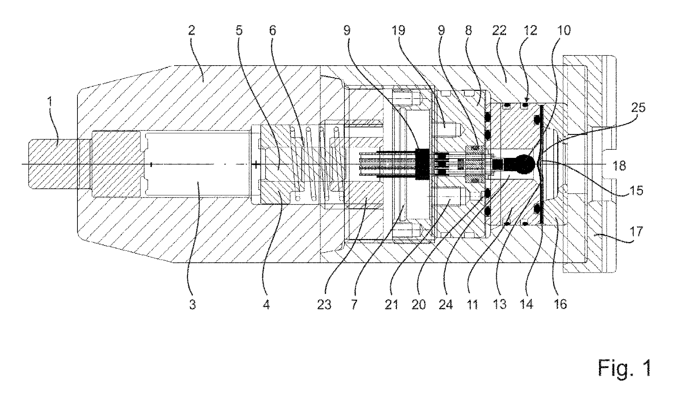

Ein grundlegender und gattungsgemäßer Aufbau ist in Figur 1 samt Legende gezeigt, wobei für die erfindungsgemäße Funktion der Injektionsvorrichtung das Gehäuse (22), sowie die Teile (7) bis (16) zu berücksichtigen sind. Die Teile (7) bis (16) können hierbei jederzeit in ein anders geformtes Gehäuse (22) eingebaut und ein Detonator, insbesondere EED (10), auch anders mit Energie versorgt werden, ohne die Funktion der Teile bzw. der Vorrichtung zu verändern. Besondere Ausführungsformen werden näher beschrieben.

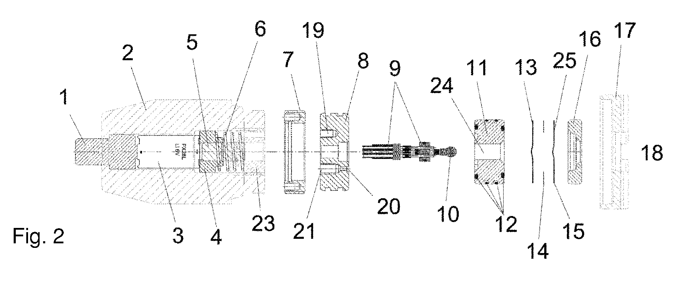

Die Geometrie der einzelnen Teile in Figur 1 können je nach Anforderung geändert oder teilweise integral zusammengefasst werden. Beispielsweise können Teile (7) bis (9) zu einem Teil integral zusammengefasst werden, oder Teil (7) kann entfallen, falls Teil (8) beispielsweise ein Außengewinde aufweist und in das Gehäuse (22) eingeschraubt wird - oder beispielsweise per Sprengring im Gehäuse (22) gehalten wird - oder ein Einlegeteil in der Spritzform des Gehäuses (22) ist (siehe ebenfalls Figur 2). Ebenfalls können die Teile (1), sowie die Teile (4) bis (6) und (23) durch die Verwendung eines herkömmlichen Tasters zur elektrischen Kontaktierung des Detonators (EED) (10) mit der Batterie (3) entfallen.

Im Rahmen dieser Erfindung wird unter "pyrotechnisches Material" ein jedes Material verstanden, welches mit einer Aktivierungsenergie zur Explosion gebracht werden kann. Dies können z.B. feste oder gasförmige Stoffe, wie Azide, Tetrazen, etc. oder anderen dem Fachmann bekannte pyrotechnische Materialien sein. Erfindungswesentlich ist, dass die Explosionsenergie eine hinreichende Impulsübertragung auf die erfindungsgemäße fixierte Doppelmembran (13, 15) erlaubt, so dass die aufgetragene Substanz (25) abgelöst, beschleunigt und auf hohe Geschwindigkeiten gebracht werden kann.

Das pyrotechnische Material ist erfindungsgemäß in einer Kammer (11) enthalten und zwar im Verbrennungsraum (24), wobei die erforderliche Aktivierungsenergie zur Erzeugung einer Explosionsenergie vorzugsweise mittels eines Detonators (10), insbesondere mittels eines Minidetonators (EED) (10) erfolgt.

Die erforderliche Aktivierungsenergie zur Explosionsauslösung in der Kammer (11) kann daher mittels einer Aktivierungseinheit erfolgen, insbesondere nicht abschließend einem Detonator (10), Anzünder, Zündstift oder Anzündstück und zwar über einen Auslösungsmechanismus mittels Reibung, Stoßen oder geeigneter Stromversorgung. Eine geeignete Aktivierungseinheit ist beispielsweise in Figur 1 dargestellt. Mittels eines Auslösers oder Tasters (1) wird die Batterie (3) gegen eine Kontaktfeder (6) und gegen einen Kontaktstift (5) gedrückt, dadurch der Stromkreis über die Anschlüsse (9) und das EED (10) geschlossen und infolge die Zündung des EEDs (10) herbeigeführt. Alternative Auslösungsmechanismen sind dem Fachmann bekannt (Reibdraht, Schlagdraht, Knackfrosch auf stoßempfindliche Anzündmischung schlagend etc.).
In einer weiteren bevorzugten Ausführungsform ist die besagte Aktivierungseinheit (1, 2, 3, 4, 5, 6, 7, 8, 9, 10) in axialer Richtung zur Kammer (11) samt Verbrennungsraum (24) ausgerichtet und der fixierten Doppelmembran (13, 15) gegenüberliegend.

Beispielsweise bei 20 bis 80 µm großen Zuckerpartikeln als aufgetragene Substanz (25) sind erfindungsgemäß Geschwindigkeiten von ca. 600 m/sec oder mehr zu erreichen. Größere Teilchen dringen wegen Ihres Impulses und des anwachsenden Verhältnisses von Dichte zum Radius deutlich einfacher in das Gewebe oder in den Körper ein, während kleinere Partikel hingegen eine deutlich höhere Geschwindigkeit als die genannten 600 m/sec benötigen.

Falls kleinere Geschwindigkeiten der durch die Doppelmembran beschleunigten Teilchen bis ca. 400 m/sec ausreichen, kann beispielsweise anstelle des Detonators 10 auch ein herkömmlicher Anzünder mit oder ohne zusätzlicher Aufladung verwendet werden.

Im Rahmen dieser Erfindung wird unter "Impuls(übertragung)" auch synonym eine Kraft-, Druckübertragung oder Beaufschlagung verstanden, welche jedenfalls ausreichend ist, die auf der hautseitigen Membran (15) der Doppelmembran (13, 15) aufgetragenen Substanz(en) (25) abzulösen und auf die gewünschten hohen Geschwindigkeiten zu beschleunigen, wodurch sie zunächst frei in Richtung des Gewebes bzw. Haut (18) oder Körper fliegen, um dort einzuschlagen, die Gewebe- oder Körperbarriere zu durchbrechen und in die gewünschte Tiefe des Gewebes, insbesondere Haut oder Körpers einzudringen.
Im Rahmen dieser Erfindung bedeutet "Haut" eine Gewebebarriere eines Menschen, Säugetier oder Tier. Die Haut übt als das größte Organ des Menschen zahlreiche lebenswichtige Funktionen aus, insbesondere die Epidermis, dient als Barriereorgan. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt. Durch die erfindungsgemäße nadellose oder nadelfreie Applikation können infolge dessen die Stoffe in den Körper lokal eintreten und ggfs. in die Blutbahnen münden

In einer weiteren Ausführungsform kann der Verbrennungsraum (24) in der Kammer (11) vorzugsweise mit einer viskosen zähflüssigen Masse versehen sein, wie Fett, Öle und dergleichen, zwecks Verkleinerung des Leerraumes im Verbrennungsraum (24) und dadurch eine Erhöhung des Druckanstieges bei kleiner Aufladung des Detonators (EED) (10) bewirken.

Die Kammer (11) weist vorzugsweise O-Ringe zur Abdichtung (12) auf. Weiterhin kann die Kammer (11) eine Entlüftung (20) samt zugehöriger Abdeckung (21) aufweisen.

Wie oben bereits ausgeführt, offenbart die gattungsmäßige zugeordnete WO 2004/071558 zwar eine Membran, jedoch keine erfindungsgemäße fixierte Doppelmembran (13, 15).
In einer bevorzugten Ausführungsform besteht die erfindungsgemäße fixierte Doppelmembran (13, 15) aus Metall oder einem Material entsprechender Härte und Duktilität, solche wie Stahl, Kunststoffen, besonders bevorzugt jedoch Titan oder Titanblech. Insbesondere Titan weist eine vorteilhafte hohe Ziehgrenze bei sehr guter Beschleunigbarkeit durch seine geringere spezifische Dichte gegenüber Stahl auf, wodurch bei relativ kleinen Drücken in der Brennkammer eine deutlich höhere Geschwindigkeit der ausbeulenden Membran erreicht wird. Hier maßgeblich ist die Beschleunigungskennzahl BK=sigma 0,2/ro.

Gegenüber einer Einfachmembran vereint hierbei die fixierte Doppelmembran eine höhere Betriebssicherheit bei Materialfehlern oder Überlastungen (falls eine Membran reißt, dringt immer noch kein Abgas nach außen) mit einer deutlich besseren Verformbarkeit, was die Energieverluste beim Verformen der Doppelmembran minimieren lässt (d.h. beispielsweise statt einer 1 mm starken Einfachmembran werden zwei Membranen mit jeweils 0,5 mm übereinandergelegt, um eine Doppelmembran auszubilden. Ebefalls andere Stärkeverhältnisse sind möglich, wie beispielsweise 0,6 mm Richtung Brennkammer und 0,4 mm Richtung Haut.

Der Begriff "fixierte Doppelmembran" (13, 15) bedeutet, dass beide Membranen - die Doppelmembran - mit einer Stützscheibe (16), insbesondere an die Kammer (11), angebracht / fixiert sind und folglich nicht beweglich also arretiert sind. Weiterhin ist bevorzugt, dass die nach innen gerichtete brennkammerseitige Membran (13) eine Dicke von 0,1 mm bis 0,6 mm aufweist, vorzugsweise 0,2 mm bis 0,6 mm, besonders bevorzugt 0,5 mm. Die nach außen gerichtete hautseitige Membran (15) weist vorzugsweise ebenfalls eine Dicke von 0,1 mm bis 0,6 mm auf, vorzugsweise 0,2 mm bis 0,6 mm besonders bevorzugt 0,5 mm. Die Membranen (13, 15) können sich ganz oder teilweise berühren.

In einer weiteren bevorzugten Ausführungsform weist jedoch die brennkammerseitige Membran (13) zur hautseitigen Membran (15) einen Abstand auf und zwar vorzugsweise von 0,2 mm bis 1,5 mm, besonders bevorzugt 1 mm. Der erforderliche Abstand kann mittels eines Abstandhalters (14) zwischen den beiden Membranen ausgelegt werden. Diese Maßnahme bewirkt eine effiziente Verhinderung des Platzens der hautseitigen Membran.

In einer weiteren besonders bevorzugten Ausführungsform ist die Doppelmembran (13, 15) zur Kammer (11) und in dessen Verbrennungsraum (24) hin ganz oder teilweise ausgewölbt, ganzflächig oder vorzugsweise im zentralen radialen Bereich um einen Kreismittelpunkt. Eine solche jeweils konkave Wölbung der jeweiligen Membranen aus Sicht der Brennkammer kann der Fachmann mittels bekannten Methoden erreichen. Die besagte erfindungsgemäße Wölbung der Doppelmembran (13, 15) erlaubt zudem eine vorteilhafte erhöhte Geschwindigkeit von 10 bis 15 % samt Fokussierung und Beschleunigung der aufgetragenen Substanzen (25) in Richtung Haut (18) - vermutlich durch Stoßwellenfokussierung.

In einer weiteren bevorzugten Ausführungsform weisen die beiden Membranen (13, 15) Stützscheiben (16) auf. Die Stützscheiben sind vorzugsweise aus Kunststoff, Messing etc., welche zur energetischen Aufnahme besonders geeignet ist. Darüber hinaus ist die Stützscheibe energetisch angepasst, vorzugsweise mit einer Verjüngung zur Haut (18) hin.

Gleichzeitig kann die Stützscheibe (16) an seiner Innenkontur mit einem weichen Kunststoff dünn überzogen sein, z.B. mit Polyethylen, um das Auslösegeräusch der Vorrichtung vorteilhaft deutlich zu reduzieren - es wird beispielsweise das knallartige Geräusch vermieden, welches entsteht, wenn eine Metallmembran auf eine metallene Stützscheibe aufschlägt.

Die Stützscheibe hat zudem auch die Aufgabe, die Membranen nach deren Druckbeaufschlagung energetisch zu verzögern und den nach der Auslösung in der Brennkammer noch wirkenden hohen Druck langdauernd abzufangen und die Membranen zu stützen.

Die vorgenannten Ausführungsformen erlauben vorteilhaft das sichere Nicht-Platzen oder Nicht-Bersten mindestens der äußeren (hautseitigen) Membran der Doppelmembran. Weiterhin wird vorteilhaft erreicht, dass die aufgebrachten Substanzen eine optimierte Impulsübertragung bzw. Beschleunigung auf sehr hohe Geschwindigkeiten bis zu 800m/sec erfahren.

Im Rahmen dieser Erfindung bedeutet "aufgebrachte Substanzen (25)", solche, die zumindest einen Stoff enthalten und beispielsweise mit Ölen u.a. an der äußeren (hautseitigen) Membran (15) fixiert sind oder durch Adhäsion anhaften. Beispielsweise können die aufgebrachten Substanzen Mittel enthalten, die ein Trocknen des Wirkstoffes auf der besagten Membran erlauben. Insbesondere sind zusätzliche Haftvermittler oder Additive geeignet und beizufügen, wie Öle oder andere vorzugsweise pharmazeutisch geeignete Hilfs- und Zusatzstoffe.

Der Begriff "Substanzen" umfasst sämtliche Mittel und Stoffe, die zur zweckmäßigen Auftragung auf die Membran geeignet sind (z.B. Pulver, Partikel etc.), einschließlich ein oder mehrerer Wirkstoffe (z.B. Arzneimittel).

In einer weiteren Ausführungsform kann die Injektionsvorrichtung einen Aufsatz (17) enthalten, welcher ergonomisch an das aufsetzende Gewebe oder Haut (18) angepasst ist. Der Aufsatz (17) hat die Aufgabe, einmal die nach der Auslösung vorgewölbte Doppelmembran sicher von der Oberfläche der anliegenden Haut abzuhalten.

Ferner können in den Aufsatz (17) eingebrachte Bohrungen oder Fräsungen die mit der Beschleunigung der Doppelmembran gleichzeitig mitbeschleunigte Luft parallel zur Oberfläche abströmen lassen und ein hydraulisches Ankoppeln der Haut an die zentrale Bohrung über der Doppelmembran verhindern, welche die anliegende Haut unzulässig mit Gasdruck belasten würde.

Weiterhin betrifft die Erfindung die Verwendung oder Anwendung von ein oder mehreren Stoffen, insbesondere Wirkstoffen, insbesondere eines Arzneimittels zur nadelfreien Applikation mittels der erfindungsgemäßen Injektionsvorrichtung nach einer der vorstehenden Ausführungsformen.

Weiterhin betrifft die Erfindung die Verwendung oder Anwendung von Stoffen, insbesondere Stoffen mit beliebiger Funktion und Eignung, solche wie Farb- oder Schmierstoffen oder Materialien mit anderen besonderen Eigenschaften zur nadellosen Einbringung in Oberflächen oder Körper technischer Gegenstände mittels der erfindungsgemäßen Injektionsvorrichtung nach einer der vorstehenden Ausführungsformen.

Daher betrifft die Erfindung eine Injektionsvorrichtung nach einer der vorstehenden Ausführungsformen zur Verwendung eines Stoffes oder eines Wirkstoffes, insbesondere Arzneimittel, zur nadelfreien Applikation.

Die folgende Beispiele und Figuren sollen die Erfindung näher erläutern, ohne jedoch diese zu beschränken.
Figur 1 zeigt einen Querschnitt der erfindungsgemäßen Injektionsvorrichtung.
Figur 2 zeigt die Bestandteile zur Herstellung der erfindungsgemäßen Injektionsvorrichtung deutlicher.

### Legende:

- 1: Auslöser, Taster
- 2: Auslösergehäuse
- 3: Batterie
- 4: Federkappe
- 5: Kontaktstift
- 6: Kontaktfeder
- 7: Nutmutter
- 8: Halter für Glasdurchführung
- 9: Glasdurchführung mit Anschlüsse
- 10: Minidetonator oder Anzündstück, EED
- 11: Brennkammer oder Kammer
- 12: O-Ring-Abdichtung der Brennkammer (kann bei einigen Ausführungsformen der Vorrichtung auch entfallen)
- 13: Brennkammerseitige Membran, fixiert zu Kammer (11)
- 14: Abstandshalter, übernimmt auch Abdichtfunktionen
- 15: Hautseitige Membran, fixiert zu Membran (13) und Abstandshalter (14)
- 16: Stützscheibe
- 17: Aufsatz
- 18: Haut oder die zu penetrierende Oberfläche (liegt auf 17 an, nicht gezeichnet)
- 19: Gewinde für Masseanschlussschraube (kann bei einigen Ausführungsformen der Vorrichtung auch entfallen)
- 20: Brennkammerentlüftung
- 21: Abdeckung, Entlüftung
- 22: Gehäuse für die Teile 7-21
- 23: Führung für Kontaktstift
- 24: Verbrennungsraum
- 25: Substanzauftragung

## Patentansprüche

1. Nadellose Injektionsvorrichtung umfassend eine Kammer (11) enthaltend ein pyrotechnisches Material, wobei die Kammer (11) an einer Austrittsöffnung eine fixierte Doppelmembran (13, 15) aufweist, wobei die hautseitige Membran (15) der Doppelmembran (13, 15) mit einer Substanzauftragung (25) versehen ist.

2. Nadellose Injektionsvorrichtung nach Anspruch 1, wobei die Doppelmembran (13, 15) mittels mindestens einer Stützscheibe (16) angebracht / fixiert ist.

3. Nadellose Injektionsvorrichtung nach Anspruch 1 oder 2, wobei die brennkammerseitige Membran (13) eine Dicke von 0,1 mm bis 0,6 mm aufweist, und / oder die hautseitige Membran (15) eine Dicke von 0,1 mm bis 0,6 mm aufweist.

4. Nadellose Injektionsvorrichtung nach einem der vorstehenden Ansprüche, wobei die brennkammerseitige Membran zur hautseitigen Membran einen Abstand von 0,2 mm bis 1,5 mm aufweist.

5. Nadellose Injektionsvorrichtung nach einem der vorstehenden Ansprüche, wobei zwischen der brennkammerseitigen Membran und der hautseitigen Membran ein Abstandshalter (14) vorgesehen ist.

6. Nadellose Injektionsvorrichtung nach einem der vorstehenden Ansprüche, wobei wenigstens die brennkammerseitige Membran, bevorzugt auch die hautseitige Membran zur Kammer (11) hin ganz oder teilweise ausgewölbt ist.

7. Nadellose Injektionsvorrichtung nach einem der vorstehenden Ansprüche, wobei eine Aktivierungseinheit (1, 2, 3, 4, 5, 6, 7, 8, 9, 10) in axialer Richtung zur Kammer (11) ausgerichtet und der fixierten Doppelmembran (13, 15) gegenüberliegend ist.

8. Nadellose Injektionsvorrichtung nach einem der vorstehenden Ansprüche enthaltend einen Aufsatz (17) mit eingebrachten Bohrungen oder Fräsungen.
